# FASCICULE DE BREVET EUROPEEN

(11) **EP 3 953 445 B1**
(45) Date de publication et mention de la délivrance du brevet: **09.09.2026**
(21) Numéro de dépôt: 20716481.5
(22) Date de dépôt: 07.04.2020
(51) Int. Cl.: C12P 39/00, C12P 5/02, C12M 1/04

(54) **PROCÉDÉ ET DISPOSITIF POUR LA PRODUCTION DE MÉTHANE**
VERFAHREN UND VORRICHTUNG ZUR HERSTELLUNG VON METHAN
METHOD AND APPARATUS FOR METHANE PRODUCTION

(30) Priorité: 08.04.2019 FR 1903721
(43) Date de publication de la demande: 16.02.2022
(73) Titulaire: Institut National des Sciences Appliquées de Toulouse, 31077 Toulouse Cedex 4 (FR); Centre National de la Recherche Scientifique CNRS, 75016 Paris (FR); Institut National de Recherche pour l'Agriculture, l'Alimentation et l'Environnement (INRAE), 75338 Paris 07 (FR); Enosis, 31100 Toulouse (FR)
(72) Inventeur: RAFRAFI, Yan, 31320 PECHABOU (FR); DUMAS, Claire, 31320 PECHBUSQUE (FR); MENGELLE, Evrard, 31190 MIREMONT (FR); DUBOS, Simon, 31320 PECHBUSQUE (FR); BOUNOUBA, Mansour, 31300 TOULOUSE (FR); DELAGNES, Delphine, 31450 AYGUESVIVES (FR); LEFEBVRE, Xavier, 32200 GIMONT (FR); SPERANDIO, Mathieu, 31810 Venerque (FR); PALMADE, Stéphane, 31100 TOULOUSE (FR); GUERRE, Vincent, 31100 TOULOUSE (FR); CONTRERAS, Viviana, 31100 TOULOUSE (FR)
(74) Mandataire: Plasseraud IP
(86) Numéro de dépôt international: PCT/EP2020/059934
(87) Numéro de publication internationale: WO 2020/208042

(56) Documents cités:
- WO-A1-2013/060331
- US-A- 4 396 402
- VIOLA CORBELLINI ET AL: "Hybrid biogas upgrading in a two-stage thermophilic reactor", ENERGY CONVERSION AND MANAGEMENT., vol. 168, 1 July 2018 (2018-07-01), GB, pages 1 - 10, XP055668410, ISSN: 0196-8904, DOI: 10.1016/j.enconman.2018.04.074
- VOELKLEIN M A ET AL: "Biological methanation: Strategies for in-situ and ex-situ upgrading in anaerobic digestion", APPLIED ENERGY, vol. 235, 1 February 2019 (2019-02-01), pages 1061 - 1071, XP085587622, ISSN: 0306-2619, DOI: 10.1016/J.APENERGY.2018.11.006
- ALITALO ANNI ET AL: "Biocatalytic methanation of hydrogen and carbon dioxide in a fixed bed bioreactor", BIORESOURCE TECHNOLOGY, ELSEVIER, AMSTERDAM, NL, vol. 196, 14 August 2015 (2015-08-14), pages 600 - 605, XP029275414, ISSN: 0960-8524, DOI: 10.1016/J.BIORTECH.2015.08.021
- KOUGIAS PANAGIOTIS G ET AL: "Ex-situ biogas upgrading and enhancement in different reactor systems", BIORESOURCE TECHNOLOGY, ELSEVIER, AMSTERDAM, NL, vol. 225, 2 December 2016 (2016-12-02), pages 429 - 437, XP029853715, ISSN: 0960-8524, DOI: 10.1016/J.BIORTECH.2016.11.124
- ARYAL NABIN ET AL: "An overview of microbial biogas enrichment", BIORESOURCE TECHNOLOGY, ELSEVIER, AMSTERDAM, NL, vol. 264, 14 June 2018 (2018-06-14), pages 359 - 369, XP085420710, ISSN: 0960-8524, DOI: 10.1016/J.BIORTECH.2018.06.013

## Description

### Domaine technique

L'invention concerne des procédés ainsi que des dispositifs pour la production de méthane *ex-situ.*

### Technique antérieure

La technologie de méthanation biologique consiste en un ou plusieurs réacteurs, contenant des micro-organismes dans un milieu liquide aqueux. Ces micro-organismes sont alimentés d'une part, par des flux de dioxyde de carbone (CO₂) à l'état gazeux, et d'autre part, par des flux d'hydrogène (H₂) également à l'état gazeux. C'est le métabolisme des micro-organismes qui permet la transformation des molécules de CO₂ et d'H₂ en molécules de méthane (CH₄) et d'eau (H₂O) par réaction de méthanation.

La méthanation permet de transformer en méthane des gaz industriels contenant du CO₂, tels que le biogaz produit par méthanisation, le syngaz produit par pyrolyse ou gazéification ou les effluents de combustion. Ces gaz peuvent ainsi être injectés dans les réseaux de gaz naturel ou être utilisés directement pour des applications telles que la mobilité.

La réaction de méthanation est exothermique.

De nombreux paramètres influencent la technologie de méthanation biologique.

Typiquement, des souches et des variants spécifiques sont développés pour améliorer les procédés de méthanation, tels que par exemple ceux décrits dans la demande de brevet internationale WO2012094538 de l'Université de CHICAGO, et plus particulièrement la souche Methanothermobacter thermautotrophicus isolée UC120910, déposée le 21 décembre 2010 à l'American Type Culture Collection (ATCC^{®}) sous le numéro d'identification de dépôt de brevet ATCC^{®} PTA-11561.

On citera également, comme paramètre influençant la technologie de méthanation biologique, la composition du milieu comprenant les micro-organismes. Typiquement, la demande EP2959003 décrit l'influence de la concentration en azote sur le taux de productivité de méthane et de la biomasse contenue dans le bioréacteur.

Il est également connu que la température, le pH ainsi que la pression ont une influence directe sur la production de méthane. Par exemple, le document EP2675904 décrit des ratios pression partielle en hydrogène/pression partielle en CO₂ spécifiques permettant d'optimiser la production de méthane. La demande WO2013/060331 décrit également que le maintien du pH dans le bioréacteur entre 7 et 8 permettrait d'augmenter la quantité de méthane obtenue.

Le débit d'alimentation de gaz est également un paramètre important. Typiquement, la demande de brevet EP2872637 décrit des débits d'injection d'hydrogène et de dioxyde de carbone dans un réacteur supérieur à 2,1 volumes de gaz par volume de milieu et par minute (vvm). Selon la présente demande, de forts débits permettent un accroissement de la productivité. Cependant, la teneur en méthane dans le mélange obtenu est faible.

Les différents états de la technique permettent majoritairement d'accroitre la productivité, c'est-à-dire la quantité de méthane produite pour un volume donné de réacteur, mais ne permettent pas d'obtenir des gaz avec une teneur en méthane très élevée, notamment une teneur en méthane de 90% ou plus. L'augmentation de la productivité se fait au détriment de la teneur en méthane. A l'inverse, certains procédés permettant d'obtenir une teneur en méthane élevée ont une faible productivité.

Cependant, la teneur en méthane obtenue est un facteur clé. Pour qu'un gaz soit injecté dans le réseau de gaz naturel, celui-ci doit en effet respecter des spécifications définies par les gestionnaires de réseau de gaz naturel. Ces spécifications varient d'un pays à l'autre et incluent de nombreux critères. Ces différents critères impliquent généralement une teneur en méthane supérieure à 95%, voire plus. De même, la norme ISO 15403, qui définit les spécifications du gaz naturel pouvant être utilisé comme gaz naturel comprimé pour les véhicules, implique une teneur en méthane élevée.

L'obtention d'une teneur en méthane élevée en sortie du procédé de méthanation biologique permet donc de limiter, voire de supprimer, le post-traitement nécessaire du gaz pour atteindre les spécifications permettant de le valoriser.

De nombreux documents de l'état de la technique décrivent également des procédés hybrides mettant en œuvre un procédé en deux étapes : un réacteur de méthanation *in situ* et un réacteur de méthanation *ex-situ.* Dans le premier réacteur se déroule les réactions de méthanisation et de méthanation. Ces étapes de méthanisation et méthanation sont réalisées dans un seul et même bioréacteur (in-*situ* biogas upgrade). Ce bioréacteur est un digesteur anaérobie qui reçoit de la matière organique, dans lequel va également être injecté de l'hydrogène. Le méthane produit et l'effluent liquide vont être injectés dans un second réacteur (ex-*situ upgrade)* dans lequel se déroule la méthanation (Hybrid biogas upgrading in a two-stage thermophilic reactor, Corbellini et al.,Energy conversion and management 168 (2008) 1-10 ).

Ces procédés mettent ainsi en œuvre, une première étape d'injection d'hydrogène lors de la méthanisation de substrats organiques pour augmenter la production de méthane, et une seconde étape de méthanation *ex-situ.* Cependant, la méthanisation implique des temps de dégradation important des substrats organiques. Il est donc nécessaire de simplifier les procédés de production de méthane mais également de les intensifier.

De surcroît la méthanisation est un procédé complexe qui implique de nombreuses interactions syntrophiques au sein de consortia composés de bactéries et d'Archea anaérobies. Or l'injection d'un surplus d'hydrogène peu affecter les performances de la méthanisation en i) bloquant en partie cette chaîne trophique à cause de l'augmentation de la pression partielle en hydrogène (pH₂) (Agneessens et al. 2018) ii) augmentant le pH du réacteur à cause de la consommation du CO2 et ainsi bloquer l'activité méthanogène (Luo et Angelidaki 2012). La régulation du pH et le contrôle fin de la quantité d'hydrogène injecté sont souvent nécessaires pour pallier ces problèmes. Or ces installations sont délicates à opérer et peuvent considérablement augmenter les coûts d'installation et d'exploitation du procédé (Angelidaki et al. 2018).

### Problème technique

Si certains procédés de l'état de la technique permettent d'atteindre une bonne productivité, la teneur en méthane obtenue est faible. Tandis que d'autres procédés permettent d'obtenir un bon enrichissement en méthane dans le mélange de gaz obtenu, la productivité est moindre. Ainsi, les solutions décrites dans l'état de la technique ne permettent pas d'obtenir à la fois une productivité élevée et une teneur en méthane élevée.

Aussi, le procédé et le dispositif selon la présente invention proposent d'augmenter la teneur en méthane du gaz pour a *minima* une productivité identique à celle de l'état de la technique du réacteur, et avantageusement d'augmenter simultanément la teneur en méthane du gaz sortant et la productivité du réacteur. De plus, le procédé selon l'invention est avantageusement plus simple à mettre en œuvre comparativement aux procédés de méthanation *in-situ* qui doivent être combinés à une première étape de méthanisation.

### Exposé de l'invention

L'invention concerne un procédé de production de méthane *ex-situ* selon la revendication 1 et un dispositif de production de méthane *ex-situ* selon la revendication 6. Il est ainsi proposé un procédé de production de méthane *ex-situ* comprenant:
- Une étape (a), dans un premier bioréacteur comprenant des micro-organismes méthanogènes dans un milieu liquide, de production d'un mélange de gaz comprenant du méthane, consistant à mettre en contact lesdits micro-organismes avec des gaz entrants choisis parmi le CO₂ et l'H₂;
- Une étape (b), dans un deuxième bioréacteur, d'enrichissement en méthane du mélange de gaz obtenu à l'étape (a), consistant à transférer, au moins en partie, du premier bioréacteur au deuxième bioréacteur, d'une part le mélange de gaz obtenu à l'étape (a), d'autre part le milieu liquide contenu dans le premier bioréacteur, de façon à augmenter la teneur en méthane dans le mélange de gaz.

De manière très avantageuse, l'étape a) du procédé selon l'invention permet d'augmenter la productivité, c'est-à-dire d'augmenter la quantité de méthane produite dans le premier bioréacteur.

Avantageusement, l'étape b) du procédé selon l'invention permet l'enrichissement de la teneur en méthane dans le mélange de gaz afin d'obtenir une teneur en méthane supérieure à 90%.

Ainsi, les étapes a) et b) du procédé selon l'invention permettent d'augmenter simultanément la teneur en méthane du gaz sortant et la productivité du réacteur.

Il est connu que la méthanation peut être réalisée *in situ* dans un digesteur de biogaz ou *ex situ* dans un réacteur externe.

Dans un système de méthanation *in-situ,* un substrat organique et de l'hydrogène supplémentaire sont ajoutés au digesteur pour produire du biogaz. Comme dans le cas de la digestion anaérobie classique, les étapes de dégradation du substrat organique fournissent des intermédiaires tels que les acides gras volatils et des précurseurs comme le dioxyde de carbone pour le processus de méthanation.

Au contraire, dans un système *ex situ* comme cela est le cas pour la présente invention, le dioxyde de carbone (provenant par exemple d'un processus de fermentation), l'hydrogène, les nutriments essentiels et les micro-organismes méthanogènes sont nécessaires et doivent être introduits. Les étapes initiales de la digestion anaérobie (hydrolyse et acidogénène) ne sont pas présentes dans un système *ex-situ* (Voelkein et al., Biological methanation : strategies for in-situ and ex-situ upgrading in anaerobic digestion, Applied Energy 235 (2019).

On entend par « méthanisation » le procédé mis en œuvre dans des méthaniseurs et qui permet de transformer la matière organique des eaux usées ou des déchets en un biogaz composé de méthane (CH₄) et de dioxyde de carbone (CO₂). La méthanisation implique quatre réactions biologiques (hydrolyse, acidogénèse, acétogénèse, méthanogénèse) assurées par plusieurs espèces microbiennes qui interagissent entre elles en constituant un réseau trophique.

On entend par « méthanation *in situ »* le procédé qui consiste à injecter de l'hydrogène directement dans le méthaniseur. La méthanation *in-situ* nécessite de contrôler avec soin la quantité d'H₂ introduite dans le digesteur, car une forte concentration / pression partielle en H₂ peut poser des problèmes: comme l'inhibition des microorganismes responsable de l'acétogénèse dans les méthaniseurs..

On entend par « méthanation *ex situ* » le procédé qui consiste à injecter de l'hydrogène et du dioxyde de carbone ou du biogaz ou du syngas (mélange de gaz contenant principalement du CO, du CO₂ et de l'H₂ et produit par pyrolyse ou gazéification) dans un réacteur indépendant.

Ainsi, on entend par « procédé de production de méthane » selon la présente invention, un procédé de méthanation *ex situ.* Le procédé consiste en effet en l'injection d'hydrogène et de dioxyde de carbone dans un réacteur indépendant.

Les étapes a) et b) du procédé selon l'invention sont des étapes de méthanation *ex-situ.* Le procédé selon l'invention est donc un procédé de méthanation ex *situ* qui est plus simple à mettre en œuvre comparativement au procédé de l'état de la technique. La méthanation *ex situ* est très intéressante car i) elle permet de ne pas perturber la méthanisation (la réaction de méthanation se déroulant dans une unité extérieure) ii) elle offre la possibilité de dissocier les conditions opératoires entre le méthaniseur et le réacteur de méthanation telles que l'utilisation de température et de pression différentes. On peut donc imposer des conditions optimales pour s'adapter aux méthanogènes hydrogénophiles (température élevée et pression élevée), la pression partielle d'hydrogène n'étant plus un problème iii) le processus biochimique est plus simple car les étapes initiales de la méthanisation telles que l'hydrolyse et l'acidogénèse ne sont pas effectuées dans le réacteur iv) le procédé est plus flexible en permettant l'utilisation d'autre sources de gaz (CO₂ industriel, syngas ...) v) le gaz sortant du réacteur de méthanation est suffisamment riche en méthane pour permettre son injection direct dans le réseau de gaz naturel.

Avantageusement encore, le procédé en deux étapes selon l'invention permet d'optimiser et d'augmenter simultanément la productivité du procédé et la teneur en méthane du gaz sortant, i.e. d'obtenir un couple productivité / teneur en méthane adéquat comparativement au procédé de méthanation *ex situ* classique (par exemple réalisé dans un bioréacteur unique).

Les gaz entrants sont de l'hydrogène (H₂) et du dioxyde de carbone (CO₂).

Le CO₂ utilisé dans le procédé selon l'invention peut être un gaz pur ou provenir d'un mélange de gaz comprenant du CO₂. De manière générale, le CO₂ peut provenir de toute source. Typiquement, le gaz pur peut-être un gaz de synthèse obtenu par tout fabricant connu de l'Homme du métier. Le procédé de production de méthane selon la présente invention peut également être alimenté en CO₂ par apport de biogaz issu de la digestion de déchets organiques dans le cadre de la méthanisation.

La méthanisation peut être, à titre illustratif, une méthanisation par voie sèche ou liquide, un procédé de méthanisation de boues, de déchets agricoles, de déchets verts, de déchets agroalimentaires et ménagers.

Le procédé de production de méthane selon la présente invention peut être alimenté en biogaz directement en sortie du digesteur de méthanisation, sans recours à une épuration particulière ou après traitement des impuretés (H₂S, Composés Organiques Volatils (COV), siloxanes, etc).

Dans le procédé selon l'invention, seul le biogaz issu de la méthanisation est utilisé. Les matières fermentescibles ou substrats organiques utilisés dans le procédé de méthanisation ne sont pas injectés dans la première étape du procédé selon l'invention.

Selon un mode de réalisation, le CO₂ provient du biogaz de décharge produit par l'enfouissement de déchets organiques.

Le CO₂ peut également provenir de gaz produits par des procédés de traitement thermochimique de la biomasse sèche ou de déchets solides (procédés de pyrolyse ou de gazéification produisant du syngas).

Le CO₂ peut également être du CO₂ fatal. Le cas échéant, le CO₂ fatal est épuré afin d'éliminer les polluants et/ou obtenir une concentration en CO₂ élevée.

Dans un mode de réalisation, l'apport en CO₂ peut consister en un mélange des différentes sources telles que précédemment décrites.

L'hydrogène utilisé dans le procédé selon l'invention peut être un gaz pur ou provenir d'un mélange de gaz comprenant de l'hydrogène. De manière générale, l'hydrogène peut provenir de toute source. Typiquement, le gaz pur peut-être un gaz de synthèse obtenu par tout fabricant connu de l'Homme du métier.

Selon un mode de réalisation, l'hydrogène peut être produit par électrolyse de l'eau à partir d'électricité soutirée au réseau électrique ou provenant d'une source de production d'électricité renouvelable.
L'électrolyse pourra être l'électrolyse alcaline, l'électrolyse PEM (à membrane), ou l'électrolyse haute température (SOEC).

Selon un mode de réalisation l'hydrogène peut être de l'hydrogène fatal. Le cas échéant, le H₂ fatal est épuré afin d'éliminer les polluants et/ou augmenter la concentration en H₂.

Selon un mode de réalisation l'hydrogène peut également provenir de gaz produits par des procédés de traitement thermochimique de la biomasse sèche ou de déchets solides (syngas).

Selon un mode de réalisation, l'hydrogène est obtenu à partir d'organismes par photosynthèse ou par fermentation en milieu sans lumière (procédé dit de « dark fermentation ») dans un réacteur séparé.

Dans un mode de réalisation, l'apport en H₂ peut consister en un mélange des différentes sources telles que précédemment décrites.

Ainsi, le dioxyde de carbone et l'hydrogène sont injectés sous forme de gaz dans le premier bioréacteur et consommés par les micro-organismes dans la phase liquide ou milieu de réaction afin de générer un mélange de gaz dans le premier bioréacteur.

On entend par « micro-organismes méthanogènes », tous les micro-organismes capables de produire du méthane, préférentiellement à partir d'hydrogène et de dioxyde de carbone.

Les organismes sont principalement des organismes hydrogénotrophes, des homoacétogènes et des méthanogènes acétoclastes.

Les méthanogènes hydrogénotrophes métabolisent directement le méthane à partir de l'hydrogène et du dioxyde de carbone. La réaction de méthanation peut être représentée de la façon suivante :

[Math. 1] 4 H₂ + CO₂ → CH₄ + 2 H₂O

Les homoacétogènes et les méthanogènes acétoclastes utilisent l'acétate comme substrat intermédiaire à la production de méthane. La réaction de méthanation est mise en œuvre selon plusieurs étapes successives et indissociables par exemple :

[Math. 2] H₂+CO₂ -> CH₃COOH CH₃COOH -> CH₄ + CO₂

Ainsi, et selon un mode de réalisation, les micro-organismes seront choisis parmi les micro-organismes méthanogènes hydrogénotrophes, les micro-organismes homoacétogènes, les micro-organismes méthanogènes acétoclastes ou un mélange de ces micro-organismes.

Les micro-organismes méthanogènes hydrogénotrophes et acétoclastes sont des Archées (ou archea) micro-organismes unicellulaires procaryotes anaérobies strictes qui appartiennent au règne des euryarchéotes. Ils pourront notamment être choisis parmi quatre classes d'archées : les Methanobacteria, les Methanomicrobia, les Methanococci et les Methanopyri.

Les micro-organismes homoacétogènes sont des bactéries (micro-organismes unicellulaires procaryotes anaérobies) qui appartiennent majoritairement à la classe des Clostridia et produisent de l'acétate à partir du CO₂ et de l'H₂.

Ces bactéries appartiennent notamment aux genres *Clostridium, Acetobacterium, Sporomusa, Acetogenium, Acetoanaerobicum, Pelobacter Butyribacterium, Eubacterium.*

Dans un mode de réalisation préférée, les micro-organismes méthanogènes sont choisis parmi les classes des Methanobacteria et des Clostridia.

On entend par les termes « biomasse » ou « culture cellulaire » l'ensemble des micro-organismes tel que précédemment définis, méthanogènes ou non, d'une ou plusieurs espèces, utilisés dans le procédé selon l'invention pour produire du méthane à partir de dioxyde de carbone et d'hydrogène. Ces micro-organismes sont placés au sein du premier réacteur par inoculation du milieu liquide contenu dans le premier réacteur ou par inoculation d'un milieu liquide qui sera par la suite introduit dans le premier réacteur selon la présente invention.

Ainsi, et avantageusement, les micro-organismes sont mis en contact avec le CO₂ et l'H₂ dans un milieu liquide dans lequel les micro-organismes sont maintenus.

Par « milieu liquide » ou « milieu de réaction », on entend le milieu liquide dans lequel les micro-organismes sont maintenus, pour générer un mélange de gaz, et dans lequel le CO₂ et l'H₂ vont être injectés et dissous, que ce milieu permette ou non la production de biomasse. Dans le contexte de l'invention, le milieu de réaction comprend au moins de l'eau, des nutriments, du CO₂ et de l'H₂ dissous.

Préférentiellement, le milieu liquide ou milieu de réaction est composé de sources de nutriments (azote, calcium, potassium, souffre, phosphore, magnésium) et des oligoéléments (fer, zinc, cuivre, cobalt, nickel, molybdène, iode et bore) nécessaires à la croissance et à l'activité microbienne.

Le milieu liquide selon l'invention pourra comprendre en outre de l'acétate.

Préférentiellement, le milieu liquide dans le premier bioréacteur est une phase liquide continue.

On entend par « phase liquide continue » au sens de la présente invention un volume liquide présentant une continuité physique, par opposition à un volume liquide discontinu constitué d'un ensemble de phases liquides sans contact les unes avec les autres tels que des gouttes liquides percolant dans une phase gaz.

On entend par « mélange de gaz » au sens de la présente invention, le mélange de gaz généré au sein du premier réacteur. Préférentiellement, le mélange de gaz comprend au moins de l'hydrogène (H₂), du dioxyde de carbone (CO₂) et du méthane (CH₄).

Avantageusement ainsi, l'étape (a) selon le procédé de l'invention permet de générer un mélange de gaz comprenant au moins du H₂, du CO₂, et du CH₄, et d'obtenir des conditions optimales pour la croissance des micro-organismes méthanogènes. Avantageusement, l'étape (a) assure une productivité élevée, grâce à un débit de gaz entrant élevé. De manière particulièrement avantageuse, le premier bioréacteur permet de convertir plus de 80% de l'hydrogène contenu dans les gaz entrants.

Avantageusement encore, l'étape (b) selon le procédé de l'invention permet de diminuer la teneur en hydrogène et en dioxyde de carbone dans le mélange de gaz obtenu à l'étape (a) et d'augmenter la teneur en méthane dans le mélange de gaz obtenu à l'étape a). Cette étape d'enrichissement est permise par l'introduction dans le deuxième bioréacteur (étape (b)) du milieu liquide prélevé dans le premier bioréacteur (étape (a)) ainsi que le transfert du mélange de gaz.

Ces deux étapes du procédé selon l'invention permettent ainsi d'optimiser et d'augmenter simultanément la productivité du procédé et la teneur en méthane du gaz sortant, i.e. d'obtenir un couple productivité / teneur en méthane adéquat.

Au sens de la présente invention, on entend par « productivité » le débit de méthane produit par volume utile de réacteur. La productivité est ainsi exprimée en Nm³/h de méthane produit par m³ utile de réacteur, unité couramment exprimée vvh par l'Homme du métier. La productivité pourra également être exprimée en NL/h de méthane produit par litre de réacteur.

La mesure de la productivité est effectuée par toute technique connue de l'Homme du métier. Typiquement, la mesure de la productivité peut être réalisée par la mesure du débit de gaz sortant total à l'aide d'une sonde Pitot et par la mesure de la teneur en méthane à l'aide d'un analyseur de gaz par spectrométrie infrarouge. Le débit de méthane calculé à partir de ces données est ramené au volume utile du réacteur mobilisé par la réaction.

Selon un mode de réalisation, la productivité est d'au moins 0,1 vvh, préférentiellement d'au moins 1 vvh, préférentiellement d'au moins 5 vvh, préférentiellement d'au moins 10 vvh, préférentiellement d'au moins 20 vvh et préférentiellement d'au moins 30 vvh.

La teneur en méthane au sens de la présente invention s'entend comme la fraction molaire du méthane au sein du gaz sortant du réacteur.

Selon un mode de réalisation, la teneur en méthane élevée s'entend d'un gaz sortant contenant au moins 80% de méthane, préférentiellement, 85%, de manière encore plus préférée 90%, préférentiellement 91%, préférentiellement 92%, préférentiellement 93%, préférentiellement 94% et de manière particulièrement préférée, au moins 95%, préférentiellement au moins 96%, préférentiellement au moins 97%, préférentiellement au moins 98%, et préférentiellement au moins 99%.

Ainsi, et avantageusement, le procédé selon l'invention permet d'obtenir du biométhane, directement injectable dans le réseau de gaz naturel, sans étape supplémentaire de purification.

La mesure de la teneur en méthane d'un gaz sortant est effectuée par toute technique connue de l'Homme du métier. Typiquement, la mesure de la teneur en méthane est effectuée à l'aide d'un analyseur de gaz par spectrométrie infrarouge ou d'une chromatographie en phase gaz. La teneur en méthane est exprimée sur gaz sec.

Avantageusement, le procédé selon l'invention permet d'obtenir une productivité d'au moins 0,15 vvh à pression atmosphérique et une teneur en méthane dans le gaz sortant d'au moins 90%.

Les caractéristiques exposées dans les paragraphes suivants peuvent, optionnellement, être mises en œuvre. Elles peuvent être mises en œuvre indépendamment les unes des autres ou en combinaison les unes avec les autres :

Selon le procédé de l'invention, le transfert du milieu liquide est réalisé par prélèvement dudit milieu liquide dans le premier bioréacteur puis par injection dudit milieu liquide dans la partie supérieure du deuxième bioréacteur afin que ledit milieu liquide circule par gravité dans le deuxième bioréacteur et soit récupéré dans le premier bioréacteur.

Préférentiellement, le milieu liquide circule, dans le second bioréacteur, à travers un garnissage ou packing. Le garnissage peut être un garnissage en vrac ou un garnissage structuré. A titre illustratif, le garnissage peut être réalisé avec des anneaux PALL^{®}.

Avantageusement, le garnissage permet d'augmenter la surface de contact entre le milieu liquide et le mélange de gaz.

Avantageusement également, le garnissage permet le développement d'un biofilm microbien. Ainsi, les catalyseurs de la réaction de méthanation peuvent être sous forme libre dans l'étape a) et sous forme de biomasse fixée dans l'étape b) du procédé selon l'invention.

Avantageusement toujours, l'injection du milieu liquide dans le deuxième bioréacteur et la circulation du milieu liquide par gravité dans ce second bioréacteur, préférentiellement à travers un garnissage, va permettre au milieu liquide de circuler par un phénomène de percolation qui va permettre d'augmenter la surface de contact entre le liquide et le mélange de gaz obtenu à l'étape (a) et de faciliter la colonisation et le renouvellement de la biomasse microbienne sur le garnissage. La teneur en hydrogène et en dioxyde de carbone du mélange de gaz obtenu à l'étape (a) va donc diminuer et le mélange de gaz va être enrichi en méthane.

Selon un mode de réalisation, dans le procédé selon l'invention, le milieu liquide est une phase liquide continue dans laquelle sont injectés les gaz entrants et le deuxième bioréacteur contient une phase gaz continue.

Avantageusement, l'injection des gaz entrant dans la phase liquide continue permet de disperser les gaz dans la phase liquide continue.

On entend par « phase gaz continue » au sens de la présente invention une phase gazeuse présentant une continuité physique, par opposition à une phase gazeuse discontinue constituée d'un ensemble de milieux gazeux sans contact les uns avec les autres tels que des bulles de gaz dispersées dans un milieu liquide.

Selon un mode de réalisation du procédé selon l'invention, le gaz obtenu à l'étape a) est transféré du premier bioréacteur au deuxième bioréacteur par la création d'un différentiel de pression entre les deux bioréacteurs.

Le gaz obtenu à l'étape a) pourra ainsi être transféré dans le deuxième bioréacteur afin d'y être enrichi selon l'étape b) du procédé.

Tout système connu de l'Homme du métier permettant la création d'un différentiel de pression entre le premier bioréacteur et le second bioréacteur pourra être utilisé. Typiquement, un compresseur pourra être utilisé.

Selon un mode de réalisation du procédé selon l'invention, du H₂ et du CO₂ peuvent en outre être injectés dans le deuxième bioréacteur.

L'injection supplémentaire de H₂ et de CO₂ dans le second bioréacteur permet de modifier la stœchiométrie des gaz afin de travailler avec des conditions stœchiométriques différentes dans les deux réacteurs. Avantageusement, le mélange de gaz injecté dans le premier réacteur présente un excès d'H₂, pour en favoriser le transfert, et du CO₂ est ajouté dans le deuxième réacteur pour assurer une teneur en méthane élevée.

L'homme du métier est en mesure d'ajuster les paramètres de température, de pression et de débit pour faire fonctionner le procédé selon l'invention.

A titre purement illustratif, la température pourra être comprise entre 50 à 70 °C, typiquement entre à 60 à 70 °C.

A titre illustratif, la pression sera comprise entre 1 et 20 bars, préférentiellement entre 2 et 18 bars, préférentiellement entre 3 et 16 bars, préférentiellement entre 4 et 14 bars, préférentiellement entre 5 et 12 bars.

L'invention concerne également un dispositif de production de méthane *ex-situ* comprenant :
- un premier bioréacteur comprenant des micro-organismes méthanogènes dans un milieu liquide ; ledit milieu liquide étant une phase liquide continue ;
- un deuxième bioréacteur comprenant une phase gaz continue et un système permettant l'augmentation des échanges gazeux ;
- un dispositif permettant d'injecter les gaz entrants dans ladite phase liquide continue contenue dans le premier bioréacteur ;
- au moins un moyen d'alimentation en milieu liquide contenu dans le premier bioréacteur coopérant avec le deuxième bioréacteur, ledit moyen étant apte à alimenter en milieu liquide le deuxième bioréacteur, ledit moyen d'alimentation comportant des moyens de pompage assurant la circulation du milieu liquide contenu dans le premier bioréacteur vers le deuxième bioréacteur, ledit milieu liquide circulant par gravité sur ledit système permettant l'augmentation des échanges gazeux, ledit milieu liquide étant récupéré dans ledit premier bioréacteur ; et;
- au moins un moyen de transfert du mélange de gaz contenu dans le premier bioréacteur vers le deuxième bioréacteur.

On entend par « dispositif de production de méthane » un dispositif de méthanation *ex situ.*

Typiquement, la circulation du milieu liquide contenu dans le premier bioréacteur vers le deuxième bioréacteur peut se faire via des moyens de pompage tels une pompe péristaltique ou tout autre moyen connu de l'homme du métier permettant d'assurer la circulation du liquide du premier bioréacteur au deuxième bioréacteur.

Préférentiellement, le moyen d'alimentation en milieu liquide est configuré pour alimenter en liquide la partie supérieure dudit deuxième bioréacteur. De manière encore plus préférée, le liquide est pulvérisé dans la partie supérieure du deuxième bioréacteur. Typiquement, la pulvérisation peut être réalisée au moyen d'un spray.

Selon un mode de réalisation, le moyen de transfert du mélange de gaz contenu dans le premier bioréacteur vers le deuxième bioréacteur est un dispositif permettant de créer un différentiel de pression entre les deux bioréacteurs.

Typiquement, ledit moyen de transfert du mélange de gaz est un compresseur.

A titre illustratif, le compresseur sera localisé en amont du premier bioréacteur et permettra de créer un différentiel de pression entre les deux bioréacteurs pour permettre le transfert du mélange de gaz obtenu dans le premier bioréacteur vers le deuxième bioréacteur. Ainsi, le gaz est transféré soit directement dans le cas où les deux réacteurs sont deux étages au sein d'un même réacteur, soit au moyen d'une canalisation si les deux réacteurs sont distincts.

Selon un mode de réalisation, le dispositif selon l'invention contient en outre un moyen d'alimentation de gaz entrants, préférentiellement H₂ et CO₂, dans le deuxième bioréacteur.

Avantageusement, cette injection supplémentaire permet de modifier la stœchiométrie des gaz.

Typiquement, ce moyen d'alimentation pourra être réalisé avec un compresseur et un débitmètre permettant d'ajuster le débit de gaz supplémentaire injecté.

Selon un mode de réalisation, le premier bioréacteur est choisi parmi une colonne à bulles, une colonne à agitation mécanique, un réacteur infiniment mélangé ou un réacteur airlift.

Selon un mode de réalisation, le dispositif permettant d'injecter les gaz entrants dans la phase liquide continue est choisi parmi les diffuseurs fines bulles tels que un diffuseur poreux de fond de colonne, un tube percé, une membrane poreuse en polymères ou en matériau céramique, un bulleur à clapet, ou parmi les contacteurs membranaires sans bulles tels que les membranes fibre creuse, ou parmi les hydroéjecteurs ou les mélangeurs statiques.

Selon un mode de réalisation, le dispositif permettant d'injecter les gaz entrants dans la phase liquide continue est un diffuseur fines bulles. Selon ce mode de réalisation, le premier bioréacteur pourra comprendre en outre un garnissage structuré. Le garnissage structuré permet de disperser les bulles.

Selon un mode de réalisation, le deuxième bioréacteur est choisi parmi un réacteur à percolation, une colonne à garnissage en vrac, une colonne à garnissage structuré, une colonne à pulvérisation, une colonne à film tombant ou une colonne à plateaux.

Selon un mode de réalisation, le système permettant l'augmentation des échanges gazeux du deuxième bioréacteur est un système de garnissage. Typiquement le système de garnissage s'entend de tout système permettant d'augmenter la surface de contact entre la phase liquide et la phase gazeuse afin d'augmenter les échanges dans le deuxième bioréacteur.

A titre illustratif, le système de garnissage peut être un garnissage en vrac constitué d'anneaux Pall^{®}, préférentiellement, d'anneaux Pall^{®} de 5/8 pouces et ayant un diamètre de 15 mm et une hauteur de 15 mm.

Préférentiellement, le premier bioréacteur est une colonne à bulles et le second bioréacteur est un réacteur à percolation.

### Brève description des dessins

D'autres caractéristiques, détails et avantages de l'invention apparaîtront à la lecture de la description détaillée ci-après, et à l'analyse des dessins annexés, sur lesquels :
**Fig. 1**

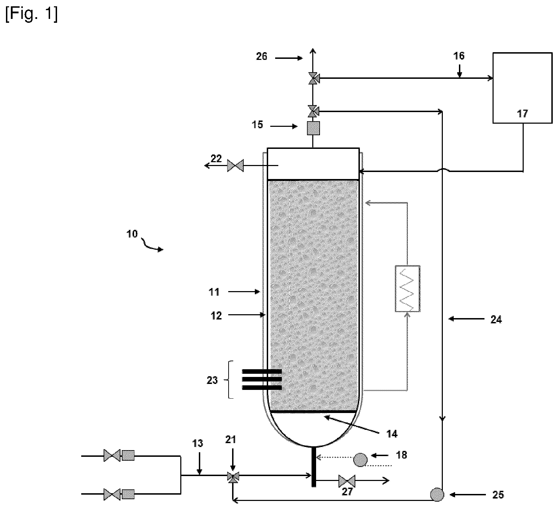

   [Fig. 1] montre un dispositif pour la production de méthane selon l'état de la technique ;
**Fig. 2**

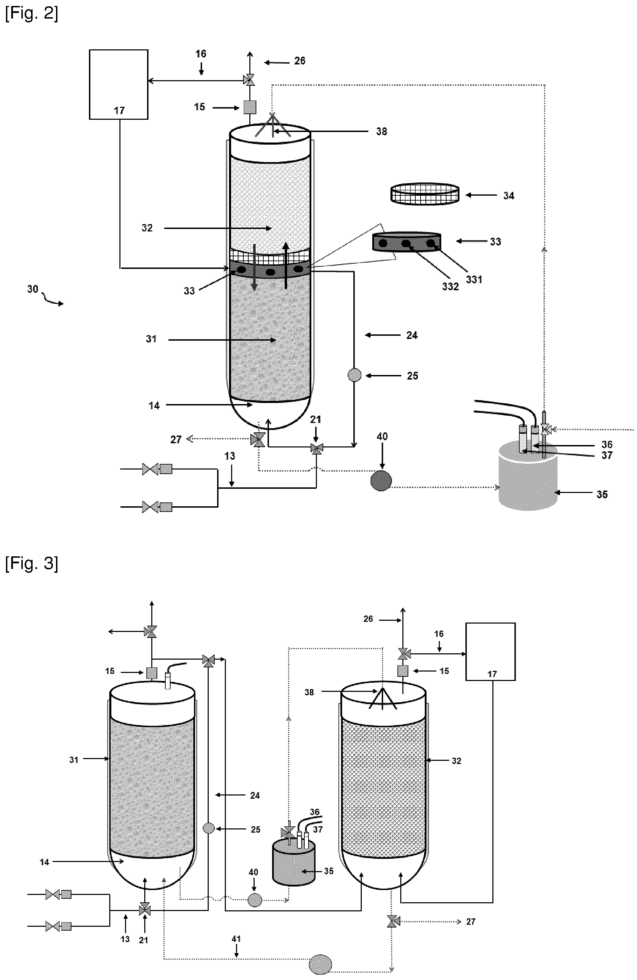

   [Fig. 2] montre un dispositif pour la production de méthane selon un mode de réalisation de l'invention dans lequel les deux bioréacteurs sont combinés (« système bi-étagé ») ;
**Fig. 3**
   [Fig. 3] montre un dispositif pour la production de méthane selon un autre mode de réalisation de l'invention dans lequel les deux bioréacteurs sont reliés par un moyen de transfert du mélange de gaz.;
**Fig. 4**

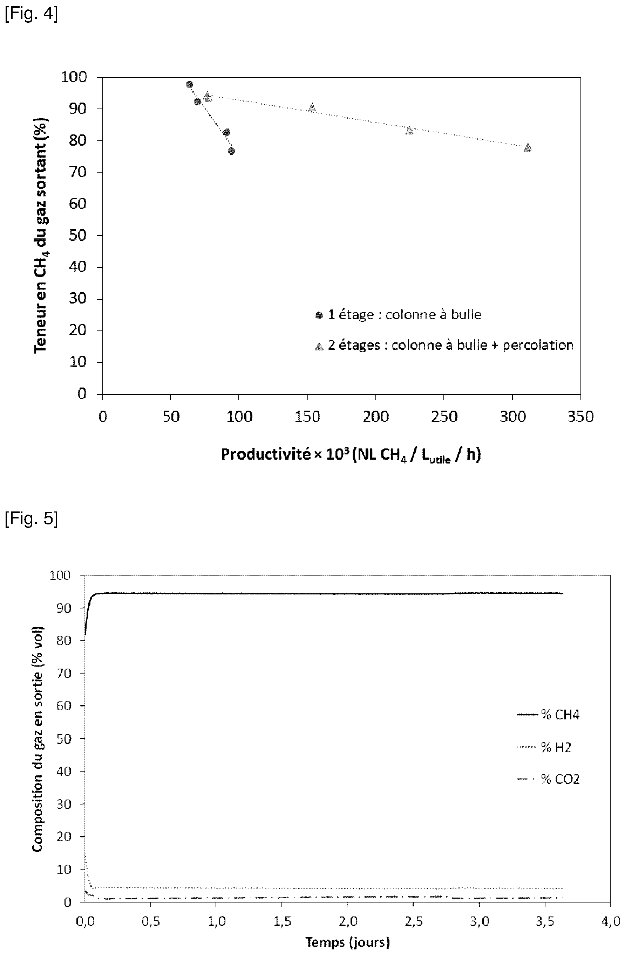

   [Fig. 4] montre une courbe représentant la productivité en NL CH₄/Lᵤₜᵢₗₑ/h en fonction de la teneur en méthane (%CH₄) d'un procédé de l'état de la technique. (« 1 étage : colonne à bulle ») et du procédé selon la présente invention (« 2 étages : colonne à bulle + percolation ») tel que représenté à la Figure 2 ;
**Fig. 5**
   [Fig.5] montre une courbe représentant la composition du gaz sortant en CH₄, CO₂, H₂ en fonction du temps du procédé selon la présente invention (« système bi étagé ») tel que représenté à la figure 5. La composition est exprimée sur gaz sec.

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Il est maintenant fait référence à la figure 1. Le dispositif 10 tel que représenté à la Figure 1 est un réacteur de méthanation de l'état de la technique permettant la production de méthane et comprenant un réacteur unique 11 à colonne à bulles de 22 litres (avec un volume utile de 18 litres), étanche aux gaz et isolé thermiquement. La température au sein du réacteur à colonne à bulles est maintenue à environ 55°C par la présence d'une chemise à circulation d'eau 12.

Un mélange de gaz entrants 13, H₂ et CO₂, est injecté dans la colonne à bulles 11 par un diffuseur à fines bulles de type fritté 14 dans la partie inférieure de la colonne à bulles 11.

La partie supérieure du réacteur est constituée d'une plaque en polychlorure de vinyle (PVC) percée de 7 orifices (non représentés) permettant le passage de sondes. Le dispositif 10 comprend également une sortie de gaz équipée d'un condenseur 15, d'une sortie de gaz 26 vers un compteur de, d'une boucle de gaz 16 reliés à des analyseurs 17 (analyse des gaz sortants permettant de quantifier respectivement, les teneurs en CO₂, H₂ et CH₄), d'une boucle de recirculation des gaz 24 de la partie supérieure du réacteur vers la partie inférieure, d'un orifice 21 de mélange des gaz recirculés avec les gaz entrants, d'une entrée 18 pour l'apport de solution nutritive, et d'une purge du milieu liquide 27. Une valve à trois voies 22 permet l'échantillonnage du gaz afin de vérifier la composition du gaz par chromatographie en phase gazeuse et pour régénérer le gaz et le milieu anoxique dans le ciel gazeux.

La concentration en dioxyde de carbone dissout, le potentiel redox ainsi que le pH sont mesurés par des sondes 23 plongées dans le milieu liquide (directement dans le réacteur). La sonde permettant la mesure du pH permet également de mesurer la température.

La colonne à bulles comprend un milieu liquide constitué de micro-organismes méthanogènes hydrogénotrophes, méthanogènes acétoclastes et homoacétogènes, de nutriments et d'oligoéléments.

Selon ce mode de réalisation de l'état de la technique, l'approvisionnement en gaz a été réalisé via deux bouteilles de gaz synthétique comprenant chacune du H₂ et du CO₂. Des débitmètres massiques permettent d'ajuster finement les débits d'entrée des gaz entrants. Une recirculation des gaz 24 du haut vers le bas de la colonne est effectuée à vitesse constante, par l'intermédiaire d'une pompe à clapet 25. Cette recirculation 24 a pour objet d'augmenter le taux de rétention gazeuse et le temps de rétention du gaz afin d'augmenter la dissolution de l'H₂ et la consommation par les micro-organismes afin de réduire la concentration en H₂ résiduel dans le mélange de gaz sortant.

Le débit du mélange de gaz sortant est mesuré par un compteur de gaz Ritter. Les gaz sortants du réacteur passent au travers d'un condenseur 15 maintenu à 4°C. L'eau de condensation est réintroduite en partie dans le réacteur afin de maintenir le volume du milieu liquide.

Le réacteur est continuellement alimenté en gaz entrant (H₂ et CO₂). En revanche, l'apport en nutriments et la purge du milieu liquide sont réalisés de manière discontinue. Le dosage du souffre est réalisé par un système de seringue à piston. Le prélèvement d'échantillons de liquide pour l'analyse des composés est réalisé dans la partie inférieure du réacteur. Typiquement, les nutriments sont injectés à partir d'une solution concentrée de nutriments, en particulier NH₄Cl à 20g/L, KH₂PO4 à 10g/L, MgCl₂ à 2g/L, CaCl₂ à 1g/L, Na₂S à 26,7 g/L et NaHCO₃ 12,4 g/L.

La composition (proportion de H₂, CO₂, CH₄) du mélange de gaz sortant est mesurée de manière continue par le prélèvement dans la partie supérieure de la colonne.

Il est maintenant fait référence à la figure 2 reproduisant un dispositif 30 pour la production de méthane selon un mode de réalisation de l'invention. Ce dispositif permet de mettre en œuvre un procédé de méthanation *ex situ.* Les éléments représentés sur la Figure 2 et portant les mêmes références que ceux de la Figure 1 représentent les mêmes objets, lesquels ne sont pas décrit de nouveaux ci-dessous.

Le dispositif ou réacteur 30 de méthanation est composé d'une colonne à bulles 31 et d'un réacteur à percolation 32, étanches au gaz et isolés thermiquement.

La colonne à bulles 31 de 22 litres (avec un volume utile de 18 litres) est reliée au réacteur à percolation 32 par une pièce 33 en PVC. La colonne à bulle et le réacteur ainsi reliés sont serrés par un collier. Le réacteur à percolation 32 est garni par des anneaux Pall^{®} de 5/8 pouces et ayant un diamètre de 15 mm et une hauteur de 15 mm (Techim France).

La colonne à bulles 31 comprend un milieu liquide comprenant des micro-organismes méthanogènes hydrogénotrophes, méthanogènes acétoclastes et homoacétogènes, des nutriments et des oligoéléments. Le milieu liquide de la colonne à bulles 31 est pompé dans la partie inférieure de la colonne à bulle 31 et est acheminé via une pompe péristaltique 40 dans la partie supérieure du réacteur à percolation 32. Le milieu liquide est injecté par pulvérisation grâce à un spray 38 présent dans la partie supérieure du réacteur à percolation. Le milieu liquide circulant par gravité sur des anneaux Pall^{®} va percoler à travers les anneaux afin d'augmenter la surface de contact entre le liquide et le gaz, jusqu'à retomber dans la colonne à bulles 31. Le mélange de gaz généré dans la colonne à bulles 31 diffuse dans le réacteur à percolation, à travers une grille en acier inoxydable 34 retenant les anneaux Pall^{®} du réacteur 32.

La productivité de méthane est élevée, notamment grâce à une pression partielle en hydrogène relativement élevée, et un débit élevé permettent ainsi une croissance microbienne élevée. Le mélange de gaz ainsi généré va diffuser, selon un différentiel de pression appliqué entre l'entrée de la colonne à bulles 31 et la sortie du réacteur à percolation 32, dans le réacteur à percolation 32 dans lequel l'hydrogène et le dioxyde de carbone vont être transformés en méthane pour atteindre une teneur élevée en méthane dans le mélange de gaz sortant. Selon ce mode de réalisation, plus de 80% de l'hydrogène contenu dans les gaz entrants est converti.

La pièce 33 permet la connexion entre la colonne à bulles 31 et le réacteur à percolation 32 et comprend des piquages, 331, 332, permettant l'analyse par chromatographie en phase gazeuse et/ou l'échantillonnage de liquide. La pièce 33 dans laquelle sont schématisés les piquage 331 et 332 est un agrandissement, sur la Figure 2, de cette pièce pour illustrer les piquages.

La concentration en dioxyde de carbone dissous, le pH et le potentiel rédox sont mesurés par des sondes 36, 37 plongées dans le liquide, soit directement dans le réacteur (non représenté), soit dans une cellule 35 connectée au réacteur tel que représentée à la Figure 2. La sonde de pH permet également la mesure de la température.

De la même façon que précédemment, l'approvisionnement en gaz est réalisé via deux bouteilles de gaz synthétique comprenant chacune du H₂ et du CO₂. Des débitmètres massiques permettent d'ajuster finement les débits d'entrée des gaz entrants. Le débit du mélange de gaz sortants est mesuré par un compteur de gaz Ritter. Les gaz sortants du réacteur passent au travers d'un condenseur maintenu à 4°C. L'eau de condensation est réintroduite en partie dans le réacteur afin de maintenir le volume du milieu liquide.

Le réacteur est continuellement alimenté en gaz entrants (H₂ et CO₂). En revanche, l'apport en nutriments et la purge du milieu liquide du réacteur sont réalisés de manière discontinue. Le dosage du souffre est réalisé par un système de seringue à piston. Le prélèvement d'échantillons de liquide pour l'analyse des composés est réalisé dans la partie inférieure du réacteur.

La composition (proportion de H₂, CO₂, CH₄) du mélange de gaz sortant est mesurée de manière continue par le prélèvement dans la partie supérieure de la colonne, par le biais des mêmes analyseurs montés en série tel que précédemment décrit.

Il est maintenant fait référence à la Figure 3. Le dispositif représenté permet également de mettre en œuvre un procédé de méthanation *ex situ.* Les éléments représentés sur la Figure 3 et portant les mêmes références que ceux de la Figure 2 représentent les mêmes objets, lesquels ne sont pas décrit de nouveaux ci-dessous.

Dans ce mode de réalisation, la colonne à bulles 31 et le réacteur à percolation 32 sont reliés entre eux par des canalisations et des pompes péristaltiques. Ce mode de réalisation permet notamment de mettre en œuvre des réacteurs de diamètres différents et de diminuer la hauteur du réacteur. Le milieu liquide contenu dans le réacteur à bulles 31 est pompé dans la partie inférieure de la colonne à bulles 31 via une pompe péristaltique 40 pour être injecté par pulvérisation au sommet du réacteur à percolation 32 grâce à un spray 38. Le milieu liquide, de la même façon que dans le mode de réalisation décrit à la Figure 2 va circuler par percolation sur les anneaux Pall^{®} et sera collecté et réinjecté par le tube 41 dans la colonne à bulles 31. Le mélange de gaz sortant généré dans la colonne à bulles 31 est transféré dans le réacteur à percolation 32 par application d'un différentiel de pression entre les deux bioréacteurs, si nécessaire à l'aide d'un compresseur.

De la même façon que précédemment, l'approvisionnement en gaz est réalisé via deux bouteilles de gaz synthétique comprenant chacune du H₂ et du CO₂. Des débitmètres massiques permettent d'ajuster finement les débits d'entrée des gaz entrants. Le débit du mélange de gaz sortants est mesuré par un compteur de gaz Ritter. Les gaz sortants du réacteur passent au travers d'un condenseur maintenu à 4°C. L'eau de condensation est réintroduite en partie dans la colonne à bulles 31 afin de maintenir le volume du milieu liquide.

Le réacteur est continuellement alimenté en gaz entrant (H₂ et CO₂). En revanche, l'apport en nutriments et la purge du milieu liquide du réacteur sont réalisés de manière discontinue. Le dosage du souffre est réalisé par un système de seringue à piston. Le prélèvement d'échantillons de liquide pour l'analyse des composés est réalisé dans la partie inférieure de la colonne à bulles 31.

La composition (proportion de H₂, CO₂, CH₄) du mélange de gaz sortant est mesurée de manière continue par le prélèvement dans la partie supérieure de la colonne, par le biais des mêmes analyseurs montés en série tels que précédemment décrit.

### Exemples

D'autres avantages, buts et caractéristiques particulières de la présente invention ressortiront des exemples qui vont suivre, faits dans un but explicatif et nullement limitatif.

Dans les exemples qui vont suivre, les différents paramètres ont été mesurés par les techniques ci-après détaillées :

Mesure de la productivité vis-à-vis du méthane produit La productivité vis-à-vis du méthane produit se calcule à l'aide de la mesure suivante : ${P}_{{CH}_{4}} = \frac{{%CH}_{4} out \times {Q}_{g , out \left(sec\right)}}{{V}_{utile}}$
Avec : PCH₄ ×10³= Productivité de méthane en NL de CH₄ / L de volume utile /h %CH₄out = pourcentage de méthane dans le gaz sortant exprimé sur gaz sec Qgout = débit de gaz sortant en NmL/h exprimé en gaz sec
Vᵤₜᵢₗₑ = volume utile du réacteur dans lequel a lieu la réaction

### Mesure de la concentration en biomasse microbienne (MVS)

La mesure de la concentration en biomasse est estimée une fois par semaine par mesure des matières volatiles en suspension (MVS) selon la norme Afnor NF T90-105-2.
Le principe consiste à effectuer un prélèvement de volume connu d'échantillon (75 mL dans notre cas). Après centrifugation pendant 15 minutes, à 13 200 rpm et à 4°C, le culot est introduit dans une coupelle en aluminium préalablement séchée et pesée. La coupelle est ensuite placée dans une étuve à 105°C pendant 24 heures. L'eau ainsi évaporée, il ne reste dans la coupelle que les matières en suspension (MES). La coupelle est alors pesée après refroidissement dans un dessiccateur. La différence de masse entre la coupelle vide et la coupelle après son passage à l'étuve correspond donc aux MES contenues dans l'échantillon. En considérant le volume de liquide initial, la mesure est exprimée en g.L⁻¹. La coupelle est ensuite mise au four à 550°C pendant 2 heures. Après refroidissement la coupelle, qui ne contient plus que les matières minérales, est de nouveau pesée. La masse de MVS est obtenue par différence entre la masse de MES et la masse des matières minérales.

### Mesure du volume de gaz sortant

Le volume de gaz sortant est mesuré par volumétrie à l'aide d'un compteur de gaz à tambour de marque Ritter^{®} (TG 05 Modèle 5). Le volume est exprimé en gaz sec.

### Mesure des teneurs en H₂, CO₂ et CH₄

La composition en H₂, CO₂, CH₄ du gaz sortant est mesuré à l'aide de différents analyseurs montés en série :
H₂ est mesuré par conductivité thermique grâce à analyseur Rosemount^{®} Binos 100 2M.
CO₂ et CH₄ sont mesurés par un analyseur de gaz non dispersifs dans l'infrarouge (NDIR) par l'utilisation de l'analyseur X-stream de Rosemount^{®}.

### Mesure du pH et de la température

Le pH et la température sont mesurés à l'aide d'une sonde et d'un transmetteur Mettler Toledo^{®}.

### Mesure du couple productivité / teneur en méthane

La productivité vis-à-vis du méthane produit est calculée par la formule ci-avant détaillée et la teneur en CH₄ est mesurée à l'aide de l'analyseur adéquat. Une fois ces informations obtenues pour différents points de fonctionnement, des graphiques représentant la teneur en CH₄ en fonction de la productivité ont été tracés et tels que représentés aux Figures 4 et 5.

Conditions de fonctionnement
Température : 52 à 57 °C
Pression : pression atmosphérique
Le débit de gaz entrant a varié de 6,3 à 43,6 NL/h.

### Exemple 1 : Evaluation de l'enrichissement en biogaz et de la productivité selon un procédé de l'état de la technique

Dans le présent exemple, le bioréacteur utilisé est le bioréacteur tel que décrit à la Figure 1.

Le bioréacteur a été inoculé à partir de micro-organismes (biomasse) issues de méthaniseurs de déchets organiques. Après croissance de la biomasse, la concentration en micro-organismes a ensuite été régulée autour de 3 g/L (MVS), par des purges de liquide et de biomasse régulières et appropriées.

Le pilote a été alimenté en continu par des gaz de synthèse, H₂ et CO₂. Le rapport entre les débits d'H₂ et de CO₂ a été maintenu constant et le débit total a progressivement augmenté avec les performances du procédé.

Le réacteur a fonctionné durant 300 jours et les performances du bioréacteur ont été évaluées quant à :
- La teneur en méthane dans le ciel gazeux ;
- La productivité de méthane en NL de CH₄ par L de réacteur par heure (PCH₄ NL/Lreac/h).

Les résultats sont présentés dans le tableau ci-après pour différents débits de gaz entrants :

**[Tabl. 1]**

| Q_{gin} (NL/h) | Q_{gout} (sec) (NL/h) | %CH₄ | PCH₄×10³ (NL/Lᵤₜᵢₗₑ/h) |
|---|---|---|---|
| 6,4 | 1,2 | 97,7 | 63,9 |
| 7,4 | 1,4 | 92,3 | 69,4 |
| 9,4 | 2,0 | 82,2 | 91,4 |
| 9,4 | 2,2 | 76,8 | 94,8 |

Q_{gin} (NL/h) correspondant au débit d'hydrogène et de dioxyde de carbone entrant en NL/h et Q_{gout} (NL/h) correspondant au débit des gaz sortant en NL/h, exprimé sur gaz sec.

### Exemple 2 : Evaluation de l'enrichissement en biogaz et de la productivité selon le procédé et dispositif de l'invention

Dans le présent exemple, le bioréacteur utilisé est le bioréacteur tel que décrit à la Figure 2.

Le bioréacteur a été inoculé à partir de micro-organismes (biomasse) issues de méthaniseurs de déchets organiques. Après croissance de la biomasse, la concentration en micro-organismes a ensuite été régulée autour de 3 g/L (MVS), par des purges de liquide et de biomasse régulières et appropriées.

Le pilote a été alimenté en continu par des gaz de synthèse, H₂ et CO₂. Le rapport entre les débits d'H₂ et de CO₂ a été maintenu constant (et le débit total a progressivement augmenté avec les performances du procédé).

Le réacteur a fonctionné durant 50 jours et les performances du bioréacteur ont été évaluées quant à :
- La teneur en méthane dans le ciel gazeux ;
- La productivité de méthane en mL de CH₄ / L de réacteur par heure (PCH₄ mL/Lreac/h)

Les résultats sont présentés dans le tableau ci-après pour différents débits de gaz entrant :

**[Tabl. 2]**

| Q_{gin} (NL/h) | Q_{gout} (sec) (NL/h) | %CH₄ | PCH₄×1 0³ (NL/Lᵤₜᵢₗₑ/h) |
|---|---|---|---|
| 14,5 | 2,9 | 94,5 | 76,7 |
| 14,5 | 3,0 | 93,9 | 77,9 |
| 29,2 | 6,1 | 90,8 | 153,6 |
| 43,6 | 9,7 | 83,3 | 225,0 |

Q_{gin} (NL/h) correspondant au débit d'hydrogène et de dioxyde carbone entrant en NL/h et Q_{gout} (NL/h) correspondant au débit des gaz sortant en NL/h, exprimé sur gaz sec.

Les résultats obtenus montrent qu'avec uniquement une colonne à bulle (réacteur à 1 étage), un compromis important doit être fait entre la teneur en méthane du gaz de sorti et la productivité. L'ajout d'un deuxième étage de percolation permet d'augmenter simultanément la productivité du procédé et la teneur en méthane du gaz produit. A titre d'illustration, le bioréacteur décrit à la Figure 1 permet d'obtenir une productivité de 91,4 x 10⁻⁴ NL_{CH4}/Lᵤₜᵢₗₑ/h pour une teneur en CH₄ de 82,2% en sortie de réacteur, alors que le bioréacteur décrit à la Figure 2 permet d'obtenir une productivité de 153,6 x 10⁻⁴ NL_{CH4}/Lᵤₜᵢₗₑ/h pour une teneur en CH₄ de 90,8% en sortie de réacteur, soit des productivité et teneur en CH₄ supérieures de 68,1% et 10,5% respectivement.

Avantageusement, le procédé et les dispositifs selon l'invention permettent d'augmenter simultanément la productivité du procédé et la teneur en méthane du gaz produit, et ce de manière simplifiée dans sa mise en œuvre, et notamment comparativement au procédé de l'état de la technique combinant des étapes de méthanisation et de méthanation.

## Revendications

1. Procédé de production de méthane *ex-situ* comprenant :
- Une étape (a), dans un premier bioréacteur comprenant des micro-organismes méthanogènes dans un milieu liquide, de production d'un mélange de gaz comprenant du méthane, consistant à mettre en contact lesdits micro-organismes avec des gaz entrants choisis parmi le CO₂ et l'H₂;
- Une étape (b), dans un deuxième bioréacteur, d'enrichissement en méthane du mélange de gaz obtenu à l'étape (a), consistant à transférer, au moins en partie, du premier bioréacteur au deuxième bioréacteur, d'une part le mélange de gaz obtenu à l'étape (a), d'autre part le milieu liquide contenu dans le premier bioréacteur, de façon à augmenter la teneur en méthane dans le mélange de gaz.

2. Procédé selon la revendication 1, **caractérisé en ce que** le transfert dudit milieu liquide est réalisé par prélèvement dudit milieu liquide dans le premier bioréacteur puis par injection dudit milieu liquide dans la partie supérieure du deuxième bioréacteur afin que ledit milieu liquide circule par gravité dans le deuxième bioréacteur et soit récupéré dans le premier bioréacteur.

3. Procédé selon la revendication 1 **caractérisé en ce que**, dans le premier bioréacteur, le milieu liquide est une phase liquide continue dans laquelle sont injectés les gaz entrants, et **en ce que** le deuxième bioréacteur contient une phase gaz continue.

4. Procédé selon l'une quelconque des revendications 1 à 3 **caractérisé en ce que** les micro-organismes sont choisis parmi les micro-organismes méthanogènes hydrogénotrophes, les micro-organismes homoacétogènes, les micro-organismes méthanogènes acétoclastes ou un mélange de ces micro-organismes.

5. Procédé selon l'une quelconque des revendications 1 à 4, **caractérisé en ce que** du H₂ et du CO₂ peuvent en outre être injectés dans le deuxième bioréacteur.

6. Dispositif de production de méthane *ex-situ* **caractérisé en ce qu'**il comprend :
- un premier bioréacteur comprenant des micro-organismes méthanogènes dans un milieu liquide, ledit milieu liquide étant une phase liquide continue;
- un deuxième bioréacteur comprenant une phase gaz continue et un système permettant l'augmentation des échanges gazeux;
- un dispositif permettant d'injecter les gaz entrants dans ladite phase liquide continue contenue dans le premier bioréacteur ;
- au moins un moyen d'alimentation en milieu liquide contenu dans le premier bioréacteur coopérant avec le deuxième bioréacteur, ledit moyen étant apte à alimenter en milieu liquide le deuxième bioréacteur, ledit moyen d'alimentation comportant des moyens de pompage assurant la circulation du milieu liquide contenu dans le premier bioréacteur vers le deuxième bioréacteur, ledit milieu liquide circulant par gravité sur ledit système permettant l'augmentation des échanges gazeux , ledit milieu liquide étant récupéré dans ledit premier bioréacteur ;et
- au moins un moyen de transfert du mélange de gaz contenu dans le premier bioréacteur vers le deuxième bioréacteur.

7. Dispositif selon la revendication 6 **caractérisé en ce que** le deuxième bioréacteur contient une phase gaz continue.

8. Dispositif selon l'une quelconque des revendications 6 à 7 **caractérisé en ce que** le dispositif contient en outre un moyen d'alimentation de gaz entrants dans le deuxième bioréacteur.

9. Dispositif selon l'une quelconque des revendications 6 à 8 **caractérisé en ce que** le premier bioréacteur est choisi parmi une colonne à bulles, une colonne à agitation mécanique, un réacteur infiniment mélangé ou un réacteur airlift et **en ce que** le second bioréacteur est choisi parmi un réacteur à percolation, une colonne à garnissage en vrac, une colonne à garnissage structuré, une colonne à pulvérisation, une colonne à film tombant ou une colonne à plateaux.

10. Dispositif selon l'une quelconque des revendications 6 à 9 **caractérisé en ce que** le dispositif permettant d'injecter les gaz entrants dans ladite phase liquide continue est choisi parmi les diffuseurs fines bulles, tels que un diffuseur poreux de fond de colonne, un tube percé, une membrane poreuse en polymères ou en matériau céramique, un bulleur à clapet, ou les contacteurs membranaires sans bulles tels que les membranes fibre creuse, ou un hydroéjecteur ou un mélangeur statique.

## Patentansprüche

1. Verfahren zur *Ex-situ*-Produktion von Methan, umfassend:
- einen Schritt (a), in einem ersten Bioreaktor umfassend methanogene Mikroorganismen in einem flüssigen Medium, der Produktion eines Gasgemisches umfassend Methan, bestehend aus Inkontaktbringen der Mikroorganismen mit eintretenden Gasen ausgewählt aus CO₂ und H₂;
- einen Schritt (b), in einem zweiten Bioreaktor, der Methananreicherung des in Schritt (a) erhaltenen Gasgemisches, bestehend aus wenigstens teilweisem Übertragen einerseits des in Schritt (a) erhaltenen Gasgemisches und andererseits des in dem ersten Bioreaktor enthaltenen flüssigen Mediums von dem ersten Bioreaktor in den zweiten Bioreaktor, derart, dass der Methangehalt in dem Gasgemisch gesteigert wird.

2. Verfahren nach Anspruch 1, **dadurch gekennzeichnet, dass** die Übertragung des flüssigen Mediums durch Entnahme des flüssigen Mediums in dem ersten Bioreaktor und anschließend durch Einspritzen des flüssigen Mediums in den oberen Teil des zweiten Bioreaktors erfolgt, sodass das flüssige Medium durch Schwerkraft in dem zweiten Bioreaktor zirkuliert und in dem ersten Bioreaktor zurückgewonnen wird.

3. Verfahren nach Anspruch 1, **dadurch gekennzeichnet, dass** in dem ersten Bioreaktor das flüssige Medium eine kontinuierliche flüssige Phase ist, in welche die eintretenden Gase eingespritzt werden, und dass der zweite Bioreaktor eine kontinuierliche Gasphase enthält.

4. Verfahren nach einem der Ansprüche 1 bis 3, **dadurch gekennzeichnet, dass** die Mikroorganismen ausgewählt sind aus den hydrogenotrophen methanogenen Mikroorganismen, den homoacetogenen Mikroorganismen, den acetoklastischen methanogenen Mikroorganismen oder einer Mischung dieser Mikroorganismen.

5. Verfahren nach einem der Ansprüche 1 bis 4, **dadurch gekennzeichnet, dass** H₂ und CO₂ ferner in den zweiten Bioreaktor eingespritzt werden können.

6. Vorrichtung zur *Ex-situ*-Produktion von Methan, **dadurch gekennzeichnet, dass** sie umfasst:
- einen ersten Bioreaktor umfassend methanogene Mikroorganismen in einem flüssigen Medium, wobei das flüssige Medium eine kontinuierliche flüssige Phase ist;
- einen zweiten Bioreaktor umfassend eine kontinuierliche Gasphase und ein System, welches die Steigerung von Gasaustauschen erlaubt;
- eine Vorrichtung, welche Einspritzen der eintretenden Gase in die kontinuierliche flüssige Phase erlaubt, welche in dem ersten Bioreaktor enthalten ist;
- wenigstens ein Mittel zur Zufuhr von in dem ersten Bioreaktor enthaltenen flüssigem Medium, welches mit dem zweiten Bioreaktor zusammenwirkt, wobei das Mittel dazu geeignet ist, dem zweiten Bioreaktor flüssiges Medium zuzuführen, wobei das Mittel zur Zufuhr Pumpmittel umfasst, welche die Zirkulation des in dem ersten Bioreaktor enthaltenen flüssigen Mediums zu dem zweiten Bioreaktor sicherstellen, wobei das flüssige Medium durch Schwerkraft durch das System zirkuliert, was die Steigerung von Gasaustauschen erlaubt, wobei das flüssige Medium in dem ersten Bioreaktor zurückgewonnen wird; und
- wenigstens ein Mittel zum Übertragen des in dem ersten Bioreaktor enthaltenen Gasgemisches in den zweiten Bioreaktor.

7. Vorrichtung nach Anspruch 6, **dadurch gekennzeichnet, dass** der zweite Bioreaktor eine kontinuierliche Gasphase enthält.

8. Vorrichtung nach einem der Ansprüche 6 bis 7, **dadurch gekennzeichnet, dass** die Vorrichtung ferner ein Mittel zur Zufuhr eintretender Gase in den zweiten Bioreaktor enthält.

9. Vorrichtung nach einem der Ansprüche 6 bis 8, **dadurch gekennzeichnet, dass** der erste Bioreaktor ausgewählt ist aus einer Blasensäule, einer mechanisch gerührten Kolonne, einem ideal durchmischten Reaktor oder einem Airlift-Reaktor, und dass der zweite Bioreaktor ausgewählt ist aus einem Perkolationsreaktor, einer Kolonne mit loser Packung, einer Kolonne mit strukturierter Packung, einer Sprühkolonne, einer Fallfilmkolonne oder einer Bodenkolonne.

10. Vorrichtung nach einem der Ansprüche 6 bis 9, **dadurch gekennzeichnet, dass** die Vorrichtung, welche Einspritzen der eintretenden Gase in die kontinuierliche flüssige Phase erlaubt, ausgewählt ist aus Feinblasendiffusoren, wie etwa einem porösen Kolonnenbodendiffusor, einem durchbohrten Rohr, einer porösen Membran aus Polymeren oder Keramikmaterial, einem Ventil-Sprudler oder blasenfreien Membrankontaktoren wie Hohlfasermembranen, oder einem Hydroejektor oder einem statischen Mischer.

## Claims

1. *Ex-situ* methane production method comprising:
- a step (a), in a first bioreactor comprising methanogenic microorganisms in a liquid medium, of producing a gas mixture comprising methane, consisting of bringing said microorganisms into contact with incoming gases selected from CO₂ and H₂;
- a step (b), in a second bioreactor, of enriching the gas mixture obtained in step (a) with methane, consisting of transferring, at least in part, from the first bioreactor to the second bioreactor, on the one hand, the gas mixture obtained in step (a), on the other hand, the liquid medium contained in the first bioreactor, so as to increase the methane content in the gas mixture.

2. Method according to claim 1, **characterised in that** the transfer of said liquid medium is carried out by withdrawing said liquid medium from the first bioreactor and then injecting said liquid medium into the upper part of the second bioreactor so that said liquid medium flows by gravity through the second bioreactor and is recovered in the first bioreactor.

3. Method according to claim 1, **characterised in that**, in the first bioreactor, the liquid medium is a continuous liquid phase into which the incoming gases are injected, and **in that** the second bioreactor contains a continuous gas phase.

4. Method according to any one of claims 1 to 3, **characterised in that** the microorganisms are selected from hydrogenotrophic methanogenic microorganisms, homoacetogenic microorganisms, acetoclastic methanogenic microorganisms or a mixture of these microorganisms.

5. Method according to any one of claims 1 to 4, **characterised in that** H₂ and CO₂ can further be injected into the second bioreactor.

6. *Ex-situ* methane production device, **characterised in that** it comprises:
- a first bioreactor comprising methanogenic microorganisms in a liquid medium, said liquid medium being a continuous liquid phase;
- a second bioreactor comprising a continuous gas phase and a system for increasing gas exchanges;
- a device for injecting the incoming gases into said continuous liquid phase contained in the first bioreactor;
- at least one means for supplying liquid medium contained in the first bioreactor cooperating with the second bioreactor, said means being able to supply liquid medium to the second bioreactor, said supply means including pumping means ensuring the circulation of the liquid medium contained in the first bioreactor to the second bioreactor, said liquid medium circulating by gravity on said system allowing the increase of gas exchanges, said liquid medium being recovered in said first bioreactor; and
- at least one means for transferring the gas mixture contained in the first bioreactor to the second bioreactor.

7. Device according to claim 6, **characterised in that** the second bioreactor contains a continuous gas phase.

8. Device according to any one of claims 6 to 7, **characterised in that** the device further contains a means for supplying incoming gas into the second bioreactor.

9. Device according to any one of claims 6 to 8, **characterised in that** the first bioreactor is selected from a bubble column, a mechanical stirring column, an infinitely mixed reactor or an airlift reactor, and **in that** the second bioreactor is selected from a percolation reactor, a loose packed column, a structured packed column, a spray column, a falling-film column or a tray column.

10. Device according to any one of claims 6 to 9, **characterised in that** the device for injecting the incoming gases into said continuous liquid phase is selected from fine-bubble diffusers, such as a porous column bottom diffuser, a perforated tube, a porous membrane made of polymers or ceramic material, a flap-type bubbler, or bubble-free membrane contactors such as hollow fibre membranes, or a hydroejector or a static mixer.
